(19) Europäisches Patentamt
European Patent Office
Office européen des brevets

(11) EP 0 591 204 B1

(12) # EUROPÄISCHE PATENTSCHRIFT

(45) Veröffentlichungstag und Bekanntmachung des Hinweises auf die Patenterteilung:
**08.01.1997 Patentblatt 1997/02**

(21) Anmeldenummer: **91918147.9**

(22) Anmeldetag: **11.10.1991**

(51) Int. Cl.$^6$: **A61K 47/32**, C08F 20/28, C07C 43/11, C07C 69/54, C07D 309/12

(86) Internationale Anmeldenummer:
**PCT/EP91/01933**

(87) Internationale Veröffentlichungsnummer:
**WO 92/10211 (25.06.1992 Gazette 1992/14)**

(54) **PHARMAZEUTISCHE ZUBEREITUNGSFORMEN**
PHARMACEUTICAL FORMULATIONS
FORMULATIONS DE PREPARATIONS PHARMACEUTIQUES

(84) Benannte Vertragsstaaten:
**AT BE CH DE DK ES FR GB IT LI NL SE**

(30) Priorität: **12.12.1990 DE 4039602**

(43) Veröffentlichungstag der Anmeldung:
**13.04.1994 Patentblatt 1994/15**

(73) Patentinhaber: **Bauer, Kurt H., Prof. Dr.**
**D-79112 Freiburg (DE)**

(72) Erfinder:
- **BAUER, Kurt H., Prof. Dr.**
  **D-7800 Freiburg 33 (DE)**
- **KIEFER, Markus**
  **D-7812 Bad Krozingen (DE)**

(74) Vertreter: **Grussdorf, Jürgen, Dr. et al**
**Patentanwälte Zellentin & Partner**
**Rubensstrasse 30**
**67061 Ludwigshafen (DE)**

(56) Entgegenhaltungen:
**EP-A- 0 157 928** **FR-A- 2 187 826**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.13.11/3.4

## Beschreibung

Die vorliegende Erfindung bezieht sich auf pharmazeutische Zubereitungsformen, speziell auf solche, die zur intravenösen Anwendung bestimmt sind.

Pharmazeutische Wirkstoffe, die zur intravasalen Anwendung bestimmt sind, müssen in Form wässriger Lösungen appliziert werden. Bei Wirkstoffen, die keine ausreichende Wasserlöslichkeit aufweisen, treten hierbei Probleme auf. So ist beispielsweise die Wasserlöslichkeit von Diazepam, Nifedipin, Propofol etc. zu gering, um die therapeutische Dosis in vertretbarem Volumen in Lösung zu bringen. Zur Entwicklung geeigneter Formulierungen ist man bei diesen Wirkstoffen auf die Vorwendung lösungsvermittelnder Hilfsstoffe angewiesen. Diese Hilfsstoffe müssen in Wasser löslich, oder damit mischbar sein. Sie dürfen keine pharmakologische Eigenwirkung besitzen und sollten sich durch eine möglichst geringe Toxizität auszeichnen. Allgemein unterscheidet man drei Gruppen von pharmazeutisch verwendeten Lösungsvermittlern:

(1) Wassermischbare Cosolventien (Propylenglykol, Glycerol, Ethanol, Polyethylenoxide)

(2) Molekülkomplexe/Einschlußverbindungen (Na-Salicylat, Na-Benzoat / Cyclodextrine)

(3) Amphiphile Substanzen

- Mizellare Solubilisation (Tenside, Cotenside)
- Liposomale Solubilisation (Lecithin)

Die wassermischbaren Cosolventien zeichnen sich durch ein hohes Lösungsvermögen für lipophile Wirkstoffe aus. Durch Zusatz einer nichtstöchiometrischen Menge dieser hydrophilen Lösungsmittel können in wässriger Lösung therapeutisch geeignete Konzentrationen der Wirkstoffe erreicht werden. Diese Form der Lösungsvermittlung wird gelegentlich als Hydrotropie bezeichnet. Die Verwendung dieser hydrophilen Lösungsmittel ist jedoch aus toxikologischer Sicht nicht unbedenklich. So wurde in über 50 % der Fälle eine oberflächliche Thrombophlebitis festgestellt. Intravasale hämolytische Reaktionen sind für Propylenglykol und Ethanol nachweisbar. Darüber hinaus wird eine Injektion dieser Substanzen als sehr schmerzhaft empfunden, da an der Einstichstelle Gewebeläsionen auftreten.

Bei den Molekülkomplexen reicht der lösungsvermittelnde Effekt in aller Regel nicht aus, um eine therapeutische Dosis zu lösen. Da die Liganden in äquimolaren Verhältnissen eingesetzt werden, sind sie in hoher Konzentration anwesend. Daher sind mögliche sensorische (Farbe, Geruch) und/oder pharmakologische Effekte des Liganden nicht auszuschließen. Vorteilhaft kann jedoch die physikalische Stabilität der Systeme erwähnt werden.

Cyclodextrine (CDe), die durch enzymatischen Abbau von Stärke gewonnen werden, sind Ringmoleküle die aus 6, 7 und 8 Glucoseeinheiten aufgebaut sind. Die Bezeichnung lautet $\alpha$, $\beta$, und $\gamma$-Cyclodextrin. Sie besitzen die Fähigkeit mit zahlreichen Wirkstoffmolekülen Einschlußverbindungen zu bilden. Die treibende Kraft für den Einschluß der Substanzen ist neben van der Waals-Wechselwirkungen und der Ausbildung von H-Brücken zwischen Gastmolekül und OH-Gruppen der Glucoseeinheiten, der Entropiegewinn der aus dem Hohlraum verdrängten Wassermoleküle. Das Gastmolekül muß bei Einschluß in den Hohlraum die dort befindlichen Wassermoleküle ausstossen und seinen eigene Hydrathülle abstreifen. Die Wassermoleküle werden vom umgebenden Wasser aufgenommen, gewinnen Freiheitsgrade und tragen durch Entropiezunahme zur Stabilität der Komplexe bei. Die lösungsvermittelnde Wirkung von Cyclodextrinen ist relativ hoch; in vielen Fällen ist sie der der nichtionogenen Tenside überlegen. Der limitierende Effekt ist die schlechte Wasserlöslichkeit von $\beta$-CD selbst, die bei 1.8 % liegt. $\alpha$ -CD löst sich zu 17.4 % und $\gamma$-CD zu 25.8 % in Wasser. Die toxischen Effekte von Cyclodextrinen sind nicht unbedeutend. $\beta$-CD zeigt bei parentaler Anwendung nephrotoxische Wirkung. Die hämolytische Aktivität von Cyclodextrinen liegt in der Reibenfolge $\beta>\alpha>\gamma$ -CD (Bloois et al, Acta Pharm. Techn. 33, 1987, Seite 136). Aufgrund der nicht unbedeutenden Toxizität ist bei Cyclodextrin bevorzugt eine orale Anwendung angezeigt.

Bei den amphiphilen Hilfsstoffen unterscheidet man zwei Klassen: Einerseits die mizellare Solubilisation durch tensidhaltige Systeme und andererseits die lipsomale Solubilisisation durch Doppelschichten von Lecithin- und Phospholipidmolekülen. Die Solubilisierungskapazität der tensidhaltigen Systeme liegt i der gleichen Größenordnung wie die liposomale Solubilisierung (Y. Okada et al, Chem. Pharm. Bull, 36, 1988, Seite 2176). Im Vergleich zu der der Cosolventien ist sie jedoch wesentlich niedriger, so daß diese Art der Lösungsvermittlung nur bei niedrigdosierten Wirkstoffen anwendbar ist.

Die Schwierigkeiten einer parentalen Anwendung von Tensiden oder Tensidsystemen liegt in ihrer Unverträglichkeit mit Zellmembranen. Bei in vitro Experimenten mit Erythrocyten und Tensidlösungen beobachtet man konzentrationsabhängig, Änderungen der Ionenpermeabilität der Membran bis hin zum Auftreten von Membranporen, die zur Freisetzung des Hämoglobins führen. Die Freisetzung des Hämoglobins aus dem Erythrocyten bezeichnet man als Hämolyse. Eine wichtige Voraussetzung für die Hämolyseaktivität von Tensiden ist ihre Fähigkeit sich in die Zellmembran einzulagern. Durch die Anreicherung der Tensidmoleküle in der Erythrocytenmembran entstehen infolge der

Mischmizellbildung mit Memmbranbestandteilen Poren, durch die Hämoglobin austreten kann.

Pharmazeutisch verwendbare Tenside dürfen hämolytische Eigenschaften nicht oder nur in sehr geringem Maße aufweisen. Aus diesem Grund stehen für parentale Formulierungen nur wenige amphile Substanzen zur Verfügung (Bauer et al, Pharm. Techn., Thieme Verlag 1989).

Derzeit verwendbar sind folgende Substanzen:

1) Polyoxyethylen-Polyoxypropylen-Blockpolymerisate (Pluronic F 68 R)

2) Ethoxyliertes Ricinusöl (Cremophor EL R)

3) Polyoxyethylen-660-12-Hydroxystearat (Solutol HS 15 R)

4) Mischmizellen aus Gallensäuresalz und Lecithin

Die Blockpolymerisate auf PEO-PPO-Basis zeigen keine hämolytische Aktivität. Ihre solubilisierenden Eigenschaften sind jedoch sehr gering und somit zur Herstellung von Solubilisaten nicht geeignet. Man verwendet sie bei der Stabilisierung von Fettemulsionen zur parentalen Ernährung (Hagers Handbuch der Pharm. Praxis, Springer Verlag 1971).

Ethoxyliertes Ricinusöl ist ein wesenlich besserer Solubilisator, dem eine eindeutige Strukturformel nicht zuzuordnen ist. Das Produkt ist das nicht weiter gereinigte Gemisch aller, durch die Umsetzung von Ricinusöl mit Ethylenoxid erhältlichen Reaktionsprodukte. Es zeigt so gut wie keine hämolytische Aktivität, besitzt aber andere ernstzunehmende Nebenwirkungen. In einigen Fällen wurde nach Injektion der Substanz über allergische Reaktionen, vom Soforttyp (anaphylaktische Reaktionen) berichtet.

Auch Polyoxyechylen-12-hydroxystearat zeigt im Tierversuch am Hund leider anaphylactoide Reaktionen, die jedoch geringer sind als bei ethoxyliertem Ricinusöl. Die solubilisierenden Eigenschaften der Substanz sind gut. Als weiterer Mangel muß die, wenn auch schwache, hämolytische Aktivität angeführt werden.

Gallensäure-Lecithin-Mischmizellen sind Mischsysteme bestehend aus Phospholipiden und natürlichen Gallensäuresalzen. Sie, solubilisieren weniger gut als die nichtionischen Tenside, zeigen aber keine hämolytische Aktivität. Die hämolytisch aktiven Gallensäuren werden durch die Mischmizellbildung mit Phospholipiden quasi inaktiviert. Die Anwendung der Mischmizellen ist aufgrund ihrer aufwendigen Herstellungstechnologie etwas eingeschränkt. Ein weiteres Problem ist die Langzeitstabilität der Systeme, da Lecithin der Autoxidation unterliegt.

Die Wechselwirkungen mit biologischen Membranen, die bei Erythrocyten zur Hämolyse führen kann, ist ein Hauptproblem der Tensidanwendung bei Injektabilita. Es besteht somit ein Bedarf an amphiphilen Substanzen, die sich durch eine geringe oder gar fehlende hämolytische Aktivität auszeichnen und zugleich gute solubilisierende Eigenschaften besitzen.

Diese Aufgabe wird durch die in dem Hauptanspruch gekennzeichneten Merkmale gelöst und durch die Merkmale der Unteransprüche gefördert.

Es wurde gefunden, daß bestimmte polymere Tenside bisher unentdeckte Möglichkeiten für eine pharmazeutische Anwendung bieten. Diese Tenside sind als Monomere stark hämolytisch und/oder anaphylaktisch wirksam, überaschender Weise jedoch als Polymere mit Polymerisationsgraden von 3 oder mehr physiologisch gut verträglich, insbesondere besitzen sie keine hämolytische Aktivität. Sie sind speziell bei intravenös zu applizierenden Formulierungen anwendbar, können aber auch in Formulierungen zur oralen und rektalen Anwendung zur Absorptionsverbesserung verwendet werden.

Die vorliegende Erfindung bezieht sich dementsprechend auf pharmazeutische Formulierungen, die wenigstens einen pharmazeutischen Wirkstoff in Kombination mit einem Polymer der allgemeinen Formel I

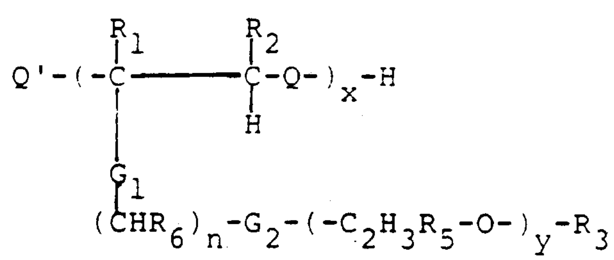

(I)

in welcher $R_1$,$R_2$,$R_5$ und $R_6$ gleich oder verschieden sind und Wasserstoff oder eine Methyl- oder Ethylgruppe darstellen,

Q eine Valenz, Sauerstoff oder eine Ester- oder Amidbrücke darstellt und Q' Wasserstoff bedeutet wenn Q eine

Valenz oder Sauerstoff darstellt und eine Hydroxyl- bzw. Aminogruppe ist, wenn Q eine Ester- bzw. Amidbrücke darstellt, x eine ganze Zahl von 3 - 50, vorzugsweise 5 - 40 ist, wenn Q eine Valenz oder Sauerstoff ist und eine ganze Zahl von 3 - 1000, vorzugsweise 50 - 100 ist, wenn Q eine Ester- oder Amidfunktion ist,

$G_1$ und $G_2$ eine Valenz, Sauerstoff, oder eine Ester- oder Amidgruppe ist, wobei die beiden Gruppen gleich oder verschieden sein können, n eine ganze Zahl von 4 - 44, vorzugsweise 12 - 16 ist,

y eine ganze Zahl von 2 - 50, vorzugsweise 10 - 40 ist und

$R_3$ Wasserstoff oder eine niedere Alkylgruppe mit 1 - 6 C-Atomen ist,

enthalten und ihre Herstellung. Ferner auf die dabei verwendeten neuen Polymere.

Im Effekt bestehen diese erfindungsgemäß zu verwendenden Polymere aus 3 Teilen, nämlich der eigentlichen Polymerkette ($-CR_1-CR_2-Q$) im folgenden als "A" bezeichnet, dem lipophilen Teil $(CHR_6)_n$ mit den beiden Verbindungsgruppen $G_1$ und $G_2$ , im folgenden als "B" bezeichnet und der hydrophilen Endgruppe $(C_2H_3R_5-O)_y-R_3$ im folgenden als "E" bezeichnet.

Die Struktur der Molekülteile A, B und E kann innerhalb weiter Grenzen variiert werden, aber das Gesamtpolymer muß wasserlöslich, pharmazeutisch akzeptabel und als wässrige Lösung stabil sein. Da das Produkt injiziert werden soll, ist es ferner erforderlich, daß es aus dem Körper über die Nieren wieder ausgeschieden werden kann, das heißt, daß das Molekül so klein ist, daß es selbst nierengängig ist, oder daß es durch die körpereigenen Enzyme in nierengängige, verträgliche Teile gespalten wird.

A ist vorzugsweise eine flexible Kette, die zweckmäßigerweise keine Ringstrukturen enthält. Ringstrukturen können jedoch in den Seitenketten vorkommen, sofern dadurch die Flexibilität von A nicht beeinträchtigt wird.

Die Struktur von A ist unkritisch und kann sowohl ein Polymerisat, ein Polykondensat als auch ein Polyaddukt darstellen. Vorzugsweise ist A ein durch Polymerisation von Vinylmonomeren insbesondere Acrylsäure, welche entsprechend mit den Gruppen B und E substituiert sind, gebildet. Die Endgruppen E sind pharmazeutisch akzeptable, hydrophile Reste.

A kann ein Oligomer, bestehend aus 3 - 50 vorzugsweise 5 bis 40, insbesondere 10 - 20 Monomereinheiten, sein. Solche Oligomere sind speziell zur intravenösen Applikation geeignet, da sie direkt durch die Niere ausgeschieden werden.

A kann aber auch eine große Anzahl von Monomereinheiten enthalten, beispielsweise 3 - 1000, vorzugsweise 50 - 100. Solche Polymere mit hohem Molekulargewicht sind zur oralen Anwendung geeignet, oder, wenn sie eine spaltbare Gruppe Q enthalten auch injizierbar.

Teil B besteht aus einer lipophilen Kohlenwasserstoffkette. Er kann gegebenenfalls auch Ringstrukturen, beispielsweise Phenylgruppen, enthalten. Die Kohlenwasserstoffkette enthält 4 - 22 C-Atome, vorzugsweise 12-16 C-Atome, um eine genügende Lipophilie zu gewährleisten.

Teil B kann mit dem Polymer A über verschiedenartige Bindungsgruppen $G_1$ verknüpft sein, die ihrerseits die Lipophilie dieses Molekülteils nicht beeinträchtigen. So kann die Verknüpfung beispielsweise über Ester-, Ether-, Thioether- oder C-C-Bindungen erfolgen.

Teil E kann aus einer großen Zahl hydrophiler Strukturelemente bestehen, beispielsweise Zucker- oder Polyethylenglykolresten. Die Endgruppe $R_3$ ist beispielsweise eine Methoxy- der Ethoxygruppe. Die Endgruppe $R_3$ kann als besonders hydrophile Struktur auch eine Salzstruktur enthalten, beispielsweise ein Alkalisalz einer Alkylcarbonsäure oder eine quartäre Alkyl-Ammoniumgruppe.

Teil E kann mit Teil B über eine Vielzahl von Bindungsgruppen $G_2$ verknüpft sein, beispielsweise durch die Gruppen $G_1$ , die bereits oben bei der Verknüpfung von A mit B beschrieben sind, aber auch durch hydrophilere Strukturen wie Amidbindungen.

Alternativ können die Gruppen E und B auch an verschiedene C-Atome des Polymerteils angeknüpft sein, wodurch sich die tautomeren Verbindungen der Formel I' ergeben

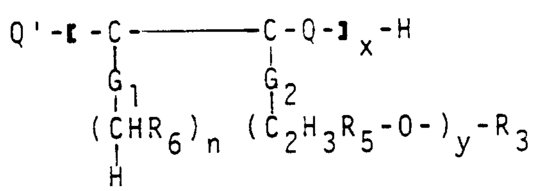

(I')

in der alle Gruppen die obige Bedeutung haben,

Der Polymerteil A kann durch herkömmliche Polymerisationstechniken hergestellt werden, beispielsweise durch

radikalische Polymerisation einer Verbindung der Formel II

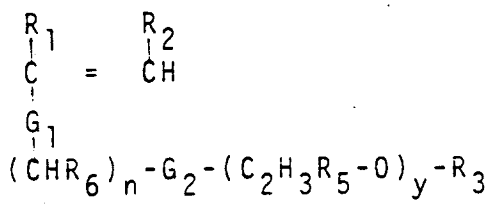

(II)

wobei ein Polymer der Formel I gebildet wird, oder durch Polymerisation einer Verbindung der Formel III

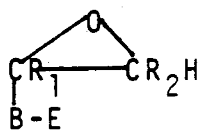

(III)

wobei Polymere erhalten werden, bei denen Q eine Ethergruppe darstellt, oder durch Polymerisation von Verbindungen IV

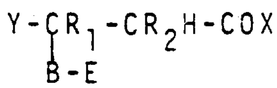

(IV)

bei denen

X eine aktivierte Gruppe und
Y eine Hydroxyl- oder Aminogruppe darstellt, wodurch Polymere der Formel I erhalten werden, in denen Q eine Ester- bzw. Amidgruppe darstellt.

Alternativ können auch Verknüpfungen von Verbindungen

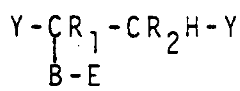

mit

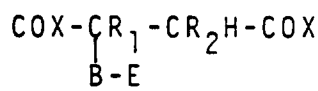

benutzt werden.

Ebenso kann das erfindungsgemäße Polymer der Formel I durch die Anknüpfung der Teile E und/oder B und E an ein geeignetes Polymerrückgrat der Formel A[Z]m erfolgen, worin "Z" eine in der Gruppe $G_1$ austauschbare Gruppe darstellt.

Die Polymerisation kann als radikalische Polymerisation, Polykondensation oder Polyaddition erfolgen.

Bevorzugte Verbindungen sind Polymere des Monomeren mit der Formel IIa

$$CH_2{=}C(R_1)\text{-}CO\text{-}O\text{-}X(OCH_2CH_2)_y\text{-}OR_2 \quad \text{(IIa)}$$

worin

$R_1$ Wassertoff oder Methyl,
$R_2$ Methyl oder Ethyl,
X $-(CH_2)_z-$, wobei z ganzzahlige Werte von 4 bis 22 annehmen kann und
y ganzzahlige Werte von 2 bis 50 umfaßt

und speziell Polymere der Formel Ia

$$-[CH_2-C(R_1)]_n- \quad \text{mit } C \text{ gebunden an } CO-O-X-(OCH_2CH_2)_y-OR_2 \qquad (Ia)$$

worin $R_1$ , $R_2$, X und y das oben definierte bedeuten und n einen Zahlenwert von 4 oder größer als 4 besitzt, und das mittlere Molekulargewicht des Polymeren kleiner als 15.000 Dalton ist.

Die vorliegende Erfindung bezieht sich ferner auf die neuen Monomeren und Polymeren; welche in den erfindungsgemäßer pharmazeutischen Formulierungen besonders geeignete Anwendungseigenschaften besitzen.

Dementsprechend bezieht sich die Erfindung auf eine Verbindung der Formel IIb

$$CH_2=C(R_1)-CO-O-X_1-(OCH_2CH_2)_8-OR_2 \qquad (IIb)$$

worin

$R_1$ Wasserstoff oder Methyl,
$R_2$ Methyl oder Ethyl und
$X_1$ $-(CH_2)_{10}-$ ist, wobei eines oder mehrere der Wasserstoffatome unabhängig voneinander durch Methyl- und/oder Ethylgruppen ersetzt sein können.

Die bevorzugten, oben erwähnten Polymere, werden durch Polymerisation einer Verbindung der Formel IIa, z.B. durch radikalische Polymerisation, hergestellt. Es werden herkömmliche Initiatorsubstanzen verwendet, wie beispielsweise Azodiisobuttersäuredinitril (AIBN). Das Molekulargewicht und der Polymerisationsgrad können durch Verwendung einer Reglersubstanz, wie beispielsweise Butanthiol, geregelt werden.

Die Monomere der Formel IIa werden nach bekannten Methoden, oder in Analogie zu bekannten Methoden, oder in Analogie zu den Methoden der nachstehenden Beispiele, hergestellt. So können beispielsweise Verbindungen der Formel IIa durch Acylierung eines entsprechenden Alkohols mit Acrylsäure- oder Methacrylsäurechlorid hergestellt werden.

Soweit die Herstellung irgend einer besonderen Ausgangssubstanz nicht in jedem Einzelfall beschrieben ist, kann diese mit herkömmlichen Methoden, oder in Analogie zu herkömmlichen Methoden, oder in Analogie zu den Methoden der nachstehenden Beispiele, hergestellt werden. Für die Herstellung sei im übrigen auf DE-A1-36 36 429, EP-A2-02 22 059, EP-A1-01 57 928 verwiesen, in denen ähnliche Verbindungen als Waschmittelkomponente und Härter beschrieben sind. Das Molekulargewicht und der Polymerisationsgrad der Polymere können mit herkömmlichen Methoden bestimmt werden.

Im allgemeinen bestehen die Polymere aus einer statistischen Mischung verschiedener Komponenten, welche sich in Molekulargewicht und Polymerisationsgrad unterscheiden. Wenn nicht anderswo ausdrücklich erwähnt, wird das Molekulargewicht und der Polymerisationsgrad durch Laserlichtstreuung (nachstehend LS) in Methanol (Blagrove R.J., Revs. Macromol. Chem. C9, 71-90, 1973), Dampfdruckosmometrie (nachstehend DDO) in Chloroform (Kamide K., Sawada M., Kobunshi Kagaku [Chem. High Polymers, Japan], 24, 751, 1967) und Gelpermeationschromatographie (nachstehend GPC, Moore J.C., Pol. Sci., A2, 835-843, 1964) mit Polystyrol als Standard und Chloroform als Lösungsmittel, bestimmt.

Die Polymere der Formel I werden mit herkömmlichen Methoden gereinigt und damit auf eine pharmazeutisch akzeptable Reinheit gebracht.

Pharmazeutische Formulierungen können durch Verarbeiten der Polymere der Formel I mit pharmazeutischen Wirkstoffen nach herkömmlichen Methoden und unter Einsatz bekannter Wirkstoffe erhalten werden.

Die verwendeten pharmazeutischen Wirkstoffe sind in Wasser schwer lösliche Substanzen, die eine Wasserlöslichkeit von höchstens 10 g/liter, oder beispielsweise von höchstens. 0,1 g/liter besitzen.

Die Wirkstoffe können aus jedem Indikationsbereich stammen, beispielsweise Antihypertensiva wie Nifedipin, Anxiolytika und Muskelrelaxantien wie Diazepam, Vitamine, wie Phytomenadion oder Mittel zur Behandlung der Hypoprothrombinämie und Anaesthetika wie Propanidid oder Propofol.

Die Solubilisierungskapazität der erfindungsgemäßen Polymere kann mit Hilfe der sogenannten Schüttelmethode bestimmt werden. Hierbei werden Lösungen unterschiedlicher Polymerkonzentration mit einem Überschuß von Beispielsweise Diazepam über einen Zeitraum von ca. 200 h bei 25 °C temperiert und periodisch geschüttelt. Das nicht gelöste Diazepam wird abfiltriert und der gelöste Anteil UV-spektrometrisch bestimmt.

Die folgenden Ergebnisse zeigen die hervorragenden Solubilisierungseigenschaften der erfindungsgemäßen Polymere.

Diazepam ohne das erfindungsgemäße Polymer

Wasserlöslichkeit = 0.005 % = 1.9 x 10-4 mol/liter

Diazepam in Gegenwart des Polymeren aus nachstehendem Beispiel 2 (hohes Molekulargewicht).

a) Repäsentativer Wert:-

Polymerkonzentration: 1.7 x 10-2 mol/liter (auf das Monomer bezogen)

Diazepamkonzentration: 1.3 x 10-3 mol/liter.

b) K Wert = 0.032 mg Diazepam pro mg Polymer (entspricht der Löslichkeitsverbesserung von Diazepam, die mit jedem mg des Polymeren erreicht wird).

Diazepam in Gegenwart des Polymeren aus nachstehendem Beispiel 3 (niederes Molekulargewicht).

a) Repräsentativer Wert:-

Polymerkonzentration: 1.7 x 10-2 mol/liter (auf das Monomer bezogen)

Diazepamkonzentration: 1.1 x 10-3 mol/liter.

b) K Wert = 0.027 mg Diazepam pro mg Polymer

Zum Vergleich: Diazepam zeigt in Gegenwart einer Lösung von 0.9 g Polyoxyethylen-660-12-hydroxystearat (Solutol HS 15$^R$, BASF AG) in 100 ml Wasser eine Löslichkeit von 7.8 x 10-4 mol/liter und einen K Wert von 0.0178 mg Diazepam pro mg Solutol.

S.Y.O. Lin et al. (Acta Pharm. Technol. (1987), 33, 222) bestimmten einen K Wert von 0.0013 mg Diazepam pro mg Pluronic F 68$^R$ (Polyoxyethylen-polyoxypropylen-Blockcopolymer).

Die Erfindung kann bei wasserlöslichen Wirkstoffen zur Bioverfügbarkeitsverbesserung verwendet werden.

Die erforderliche Menge des erfindungsgemäßen Polymeren hängt von verschiedenen Faktoren, wie der Art des Wirkstoffs, seiner Löslichkeit, seiner Wirkdosis und der beabsichtigten Applikationsart, ab. Die Menge kann mit Routinemethoden bestimmt werden.

Zur intravenösen Injektion wird die Menge des Polymeren der Formel I so gewählt, daß eine therapeutische Dosis des Wirkstoffs in einem Volumen von, beispielsweise 1 bis 5 ml flüssiger Hilfsstoffe gelöst werden kann.

Die Menge an Wirkstoff und erfindungsgemäßem Polymer kann leicht nach herkömmlichen Methoden bestimmt werden, beispielsweise durch Bioverfügbarkeitsuntersuchungen bei Tieren oder Menschen, wobei der Blutspiegel des Wirkstoffs mit Standardtechniken, wie beispielsweise Radioimmunoassay, bestimmt wird und durch pharmakologische Untersuchungen bei Tieren und Menschen, die die Bestimmung des pharmakologischen Effekts des Wirkstoffs erlauben.

Im allgemeinen werden die Polymere in Konzentrationen bis zu 30 Gewichtsprozenten, beispielsweise 0.01 bis 25 Gewichtsprozent der pharmazeutischen Formulierung, verwendet.

Die hervorragende Verträglichkeit der pharmazeutischen Formulierungen und der erfindungsgemäßen Polymere kann durch Standardtests bestimmt werden.

Es kann beispielsweise die hämolytische Aktivität wie folgt bestimmt werden.

Menschliche Erythrocyten werden aus 400 ml stabilisiertem Humanblut (stabilisiert mit einem Zusatz einer Lösung von 4.72 g Zitronensäure, 14.12 g Trinatriumcitrat und 17.92 g Glucose-Monohydrat in 1000 ml Wasser) durch mehrfaches Zentrifugieren und Waschen mit einem isotonen Phosphatpuffer pH 7.4 (Ph. Eur. III) als Suspension erhalten. Zur Herstellung einer isotonen Lösung werden die erfindungsgemäßen Polymere in der gleichen Pufferlösung gelöst. Zur Polymerlösung wird ein definiertes Volumen (0.1 ml) der Erythrocytensuspension gegeben, welche auf eine bestimmte Zellzahl eingestellt ist (beispielsweise auf 1 bis 10 x $10^9$ Zellen / ml, bestimmt durch Auszählen in der Zählkammer nach Neubauer nach Fixierung mit Hayems Reagenz, (Lösung von 0.25 g $HgCl_2$, 2.5 g $Na_2SO_4$ und, 0.5 g NaCl in 100 ml dest. Wasser). Die Mischung wird bei 37 °C mindestens eine Stunde inkubiert.

Die hämolytische Aktivität wird durch UV-spektroskopische Bestimmung der Hämoglobinkonzentration, im nach der Zentrifugation erhaltenen Überstand, bestimmt. Als Kontrollwert wird eine tensidfreie Lösung verwendet. Als Referenzwert wird eine Polyoxyethylen-p-t-octyl-phenol-Lösung (Nonidet P 40R) verwendet, die eine vollständige Lyse der Erythrocyten bewirkt.

Eine 2 prozentige (w/w) isotone Lösung des Polymers aus Beispiel 2 (hohes Molekulargewicht) zeigt nach einer Inkubationszeit von 8 h eine Hämolyseaktivität von 0.7 %, die unbedeutend ist. Die Erythrocytenkonzentration in der Reaktionslösung beträgt 1.8 x $10^8$ Erythrocyten / ml.

Eine 10 prozentige (w/w) isotone Lösung des Polymers aus Beispiel 3 (niederes Molekulargewicht) zeigt nach einer Inkubationszeit von 1 h eine Hämolyseaktivität von 0.6 %, die unbedeutend ist. Die Erythrocytenkonzentration in der Reaktionslösung beträgt 2.6 x $10^8$ Erythrocyten / ml.

Die erfindungsgemäßen pharmazeutischen Formulierungen können den spezifischen Applikationsarten, wie beispielsweise der oralen, oder externen wie der cutanen und speziell der parenteralen, beispielsweise der intravenösen Applikation, beispielsweise als Injektionslösung, angepaßt werden.

Die pharmazeutischen Formulierungen enthalten geeignete bekannte Hilfstoffe.

Intravenöse Formulierungen können auf wässriger Basis durch den Zusatz von beispielweise Zitronensäure und Dinatriumhydrogenphosphat zur pH-Wert Einstellung und isotonisierenden Zusätzen wie beispielsweise Glucose, hergestellt werden.

Die folgenden Beispiele veranschaulichen die Erfindung. Die verwendeten Abkürzungen sind auf den vorhergehenden Seiten bereits definiert worden.

BEISPIEL 1: Synthese von 11,14,17,20,23,26,29, 32,35-Nonaoxahexa-triacontanyl-methacrylat

a) 11,14,17,20,23,26,29,32,35-Nonaoxahexatriacontanyltetrahydropyranylether

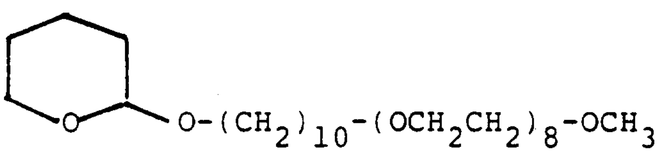

Zu einer Mischung aus 46,9 g (0,122 mol) Octaethylenglykolmonomethylether und 250 ml Toluol gibt man 2,7 g (0,12 mol) Natrium hinzu und erhitzt auf ca. 100 °C. Sobald alles Na verschwunden ist, werden bei ca. 70 °C 34,0 g (0,123 mol) 10-Chlordecanoltetrahydropyranylether zugetropft. Unter Stickstoff-Atmosphäre läßt man mehrere Tage bei 100 °C reagieren.

Das ausgefallene NaCl wird über eine G3-Fritte abgesaugt und das Filtrat mit Aceton verdünnt. Der dabei entstehende braune Niederschlag wird abzentrifugiert. Die Lösungsmittel werden entfernt.

b) 11,14,17,20,23,26,29,32,35-Nonaoxahexatriacontan-1-ol

$$HO\text{-}(CH_2)_{10}\text{-}(OCH_2CH_2)_8\text{-}OCH_3$$

Um die Schutzgruppe abzuspalten, wird der Rückstand mit 80 ml (1,37 mol) Ethanol p.a. verdünnt und mit 3,0 g (0,012 mol) PPTS (p-Pyridiniumtoluolsulfonat) 3h bei einer Temperatur von 60 °C erhitzt. Anschließend verdünnt man mit 300 ml Ether, saugt das ausgefallene PPTS ab und entfernt die Lösungsmittel. Das flüssige Produkt wird durch Kurzwegdestillation im Vakuum erhalten (Kp 220 °C, $8.10^{-3}$ HPa).

c) 11,14,17,20,23,26,29,32,35-Nonaoxahexatriacontanylmethacrylat

$$CH_2{=}C(CH_3)\text{-}CO\text{-}O\text{-}(CH_2)_{10}\text{-}(OCH_2CH_2)_8\text{-}OCH_3$$

Zu einer Lösung von 8 g (0,0148 mol) 11,14,17,20,23,26, 29,32,35 Nonaoxahexatriacontan-1-ol, 1,82 g (0,018 mol) Triethylamin in 40 ml Dichlormethan werden bei einer Temperatur von -20 °C 1,87 g (0,018 mol) Methacrylsäurechlorid und eine geringe Menge von Di-t-butylhydroxytoluol in Methylenchlorid gegeben. Es wird 12h ohne Kühlung bei RT gerührt. Das Lösungsmittel wird bei RT entfernt, das Rohprodukt flashchromatographisch über Kieselgel 60 (0,040-0,063 mm) mit Aceton/Ether 50:50 aufgearbeitet. Das Laufmittel wird bei RT abdestilliert und das Produkt als farblose Flüssigkeit erhalten.

$^1$HNMR: 1.2 - 1.8 ppm (m, 16H, $-(CH_2)_8-$), 1.9 ppm (s, 1H, HO-), 3.4 ppm (s, 3H, $-OCH_3$), 3.7 ppm (m, 34H, $-(OCH_2CH_2)_8-O-$, $-(CH_2)_8-CH_2-O-$), 4.2 ppm (m, 2H, $-COO-CH_2-CH_2-$), 5.5 ppm (s, 1H, $H-CH=C(CH_3)-COO-$), 6.1 ppm (s, 1H, $H-CH=C(CH_3)-COO-$).

IR (Film): 1700 cm-1 (C=O), 1620 cm-1 (C=C), 1110 cm-1 (C-O).

BEISPIEL 2: Polymer mit hohem Polymerisationsgrad

Eine Lösung von 4,1 g (0,0067 mol) Monomer und 0,011 g (0,000067 mol) AIBN (Azoisobuttersäurenitril) in 10 ml THF abs. wird mit $N_2$ liq. eingefroren. Durch wiederholtes Entlüften im Vakuum und Belüften mit $N_2$ mit anschließendem Auftauen wird die Lösung $O_2$-frei gemacht. Die Polymerisation erfolgt durch 15 stündiges Erwärmen auf 60 °C. Im Anschluß wird das Polymer dreimal in n-Hexan gefällt, in Dichlormethan gelöst und ca. 30 h im Hochvakuum getrocknet.

$M_w$ = 380.000 g/mol (bestimmt durch LLS); $M_w/M_n$ = 5.5 (bestimmt durch GPC); Polymerisationgrad $P_w$ = 620.

BEISPIEL 3: Polymer mit niederem Polymerisationsgrad

Die Polymerisation erfolgt unter gleichen Bedingungen wie oben mit folgendem Ansatz: 3,6 g (0,006 mol) Monomer, 0,06 g (0,00066 mol) Butanthiol und 0,01 g (0,00006 mol) AIBN in 8 ml absolutem THF.

$M_n$ = 4300 g/mol (bestimmt durch DDO); $M_w/M_n$ = 1.2 (bestimmt durch GPC); Polymerisationsgrad $P_w$ = 7.

BEISPIEL 4:

In Analogie zu Beispiel 1 wird 11,14,17,20,23,26,29-heptaoxatria-contanyl-methacrylat hergestellt und in Analogie zu Beispiel 2 polymerisiert.

BEISPIEL 5: Pharmazeutische Formulierungen

Injektionslösungen können entsprechend den folgenden Rezepturen nach herkömmlichen Methoden hergestellt werden (Mengenangaben in Gramm).

1) Propanidid-Injektionslösung

| | |
|---|---|
| Propanidid | 0,500 |
| Polymer aus Beispiel 3 | 1,500 |
| Citronensäure anh. | 0,016 |
| Dinatriumhydrogenphosphat anh. | 0,036 |
| Glucose | 0,355 |
| Wasser für Injektionszwecke ad | 10 ml |
| pH-Wert = 6,0. | |

2) Diazepam-Injektionslösung

| | |
|---|---|
| Diazepam | 0,0100 |
| Polymer aus Beispiel 3 | 0,3800 |
| Citronensäure anh. | 0,0019 |
| Dinatriumhydrogenphosphat anh. | 0,0080 |
| Glucose | 0,0675 |
| Wasser für Injektionszwecke ad | 2 ml |
| pH-Wert = 6,9 | |

3) Phytomenadion-Injektionslösung

| | | |
|---|---|---|
| Phytomenadion (Vit. K3) | | 0,0020 |
| Polymer aus Beispiel 3 | | 0,0600 |
| Citonensäure anh. | | 0,0016 |
| Dinatriumhydrogenphosphat anh. | | 0,0036 |
| Glucose | | 0,0373 |
| Wasser für Injektionszwecke | ad | 1 ml |
| pH-Wert = 6,0 | | |

4) Propofol-Injektionslösung

| | | |
|---|---|---|
| Propofol | | 0,200 |
| Polymer aus Beispiel 3 | | 2,300 |
| Citronensäure anh. | | 0,019 |
| Dinatriumhydrogenphosphat anh. | | 0,080 |
| Glucose | | 0,710 |
| Wasser für Injektionszwecke | ad | 20 ml |
| pH-Wert = 6,9 | | |

Diese Formulierungen können für die gleichen Indikationen mit den gleichen Wirkstoffmengen, wie die handelsüblichen wirkstoffgleichen Zubereitungen verwendet werden. So kann die Beispielsformulierung 2 bei der Behandlung von akuten Angstzuständen oder Muskelspasmen als intravenöse Injektion alle 4 Stunden gegeben werden.

## Patentansprüche

1. Pharmazeutische Zubereitungsform enthaltend einen in Wasser schwerlöslichen pharmazeutischen Wirkstoff und einen Lösungsmittler, **dadurch gekennzeichnet, daß** der Lösungsvermittler ein Polymeres der allgemeinen Formel I ist,

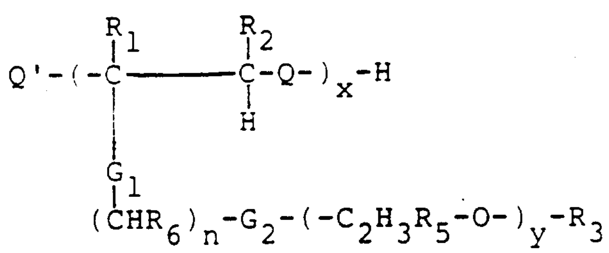

(I)

in welcher $R_1$,$R_2$,$R_5$ und $R_6$ gleich oder verschieden sind und Wasserstoff oder eine Methyl- oder Ethylgruppe darstellen,

Q eine Valenz, Sauerstoff oder eine Ester- oder Amidbrücke darstellt und Q' Wasserstoff bedeutet wenn Q eine Valenz oder Sauerstoff darstellt und eine Hydroxyl- bzw. Aminogruppe ist, wenn Q eine Ester- bzw. Amidbrücke darstellt,

x eine ganze Zahl von 3 - 50, vorzugsweise 5 - 40 ist, wenn Q eine Valenz oder Sauerstoff ist und eine ganze Zahl von 3 - 1000, vorzugsweise 50 - 100 ist, wenn Q eine Ester- oder Amidfunktion ist,

$G_1$ und $G_2$ eine Valenz, Sauerstoff, oder eine Ester- oder Amidgruppe ist, wobei die beiden Gruppen gleich oder verschieden sein können, n eine ganze Zahl von 4 - 44, vorzugsweise 12 - 16 ist,

y eine ganze Zahl von 2 - 50, vorzugsweise 10 - 40 ist und

$R_3$ Wasserstoff oder eine niedere Alkylgruppe mit 1 - 6 C-Atomen ist,

oder das Polymer die Formel I' hat

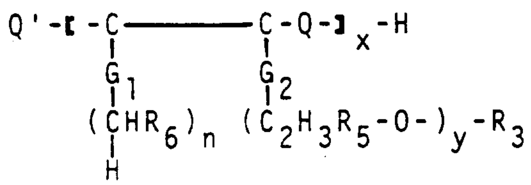

(I')

in der alle Gruppen die obige Bedeutung haben.

2. Pharmazeutische Zubereitung gemäß Anspruch 1, **dadurch gekennzeichnet, daß** das Polymer ein Polymer der Formel I'' ist,

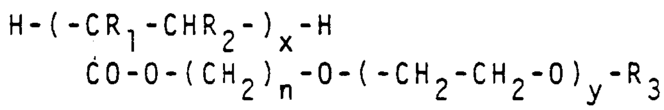

(I'')

in der $R_1$, $R_2$, $R_3$, x, n, und y die vorsehende Bedeutung, haben.

3. Pharmazeutische Zubereitung gemäß Anspruch 2, **dadurch gekennzeichnet, daß** $R_1$, $R_3$ und x die vorstehende Bedeutung haben, $R_2$ Wasserstoff ist, n gleich 10 und y gleich 8 ist.

4. Pharmazeutische Zubereitung gemäß einem der Ansprüche 1 bis 3, **dadurch gekennzeichnet, daß** x gleich 7 ist.

5. Pharmazeutische Zubereitung gemäß Anspruch 1, **dadurch gekennzeichnet, daß** das Polymer ein Polymeres der Formel I'' ist,

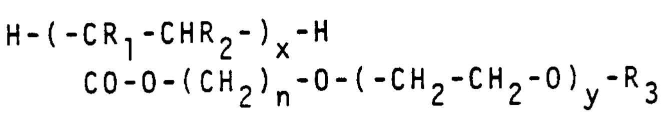

(I'')

in welcher $R_1$,$R_2$,$R_5$ und $R_6$ gleich oder verschieden sind und Wasserstoff oder eine Methyl - oder Ethylgruppe darstellen,

x eine ganze Zahl von 3 - 50, vorzugsweise 5 - 40 ist, wenn Q eine Valenz oder Sauerstoff ist und eine ganze

Zahl von 3 - 1000, vorzugsweise 50 - 100 ist, wenn Q eine Ester- oder Amidfunktion ist,

$G_1$ und $G_2$ eine Valenz, Sauerstoff, oder eine Ester- oder Amidgruppe ist, wobei die beiden Gruppen gleich oder verschieden sein können, n eine ganze Zahl von 4 - 44, vorzugsweise 12 - 16 ist,

y eine ganze Zahl von 2 - 50, vorzugsweise 10 - 40 ist und

$R_3$ Wasserstoff oder eine niedere Alkylgruppe mit 1 - 6 C-Atomen ist,

oder das Polymer die Formel I''' hat

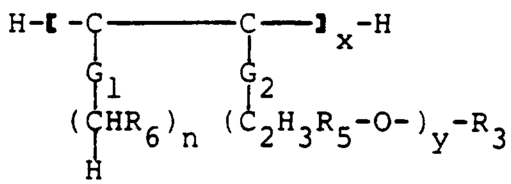

(I''')

in der alle Gruppen die obige Bedeutung haben.

6. Pharmazeutische Zubereitung gemäß Anspruch 5,**dadurch gekennzeichnet, daß** $R_1$, $R_3$ und x die vorstehende Bedeutung haben, $R_2$ Wasserstoff ist, n gleich 10 und y gleich 8 ist.

7. Pharmazeutische Zubereitung gemäß der Ansprüche 5 und 6, **dadurch gekennzeichnet, daß** x gleich 7 ist.

8. Pharmazeutische Zubereitung **dadurch gekennzeichnet, daß** der Lösungsvermittler 11,14,17,20,23,26,29,32,35,-Nonaoxahexatriacontan-1-ol oder seinen Tetrahydropyranylether beinhaltet.

9. Verwendung von Polymeren der Formel I

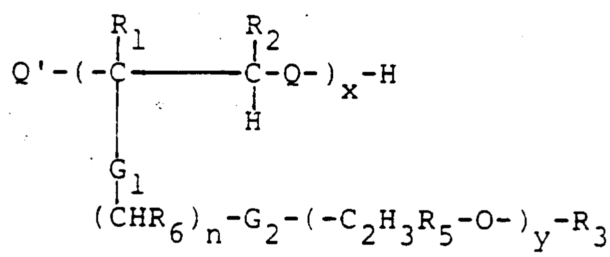

(I)

in welcher $R_1$,$R_2$,$R_5$ und $R_6$ gleich oder verschieden sind und Wasserstoff oder eine Methyl- oder Ethylgruppe darstellen,

Q eine Valenz, Sauerstoff oder eine Ester- oder Amidbrücke darstellt und Q' Wasserstoff bedeutet wenn Q eine Valenz oder Sauerstoff darstellt und eine Hydroxyl- bzw. Aminogruppe ist, wenn Q eine Ester- bzw. Amidbrücke darstellt,

x eine ganze Zahl von 3 - 50, vorzugsweise 5 - 40 ist, wenn Q eine Valenz oder Sauerstoff ist und eine ganze Zahl von 3 - 1000, vorzugsweise 50 - 100 ist, wenn Q eine Ester- oder Amidfunktion ist,

$G_1$ und $G_2$ eine Valenz, Sauerstoff, oder eine Ester- oder Amidgruppe ist, wobei die beiden Gruppen gleich oder verschieden sein können, n eine ganze Zahl von 4 - 44, vorzugsweise 12 - 16 ist,

y eine ganze Zahlt von 2 - 50, vorzugsweise 10 - 40 ist und

$R_3$ Wasserstoff oder eine niedere Alkylgruppe mit 1 - 6 C-Atomen ist,

oder das Polymer die Formel I' hat

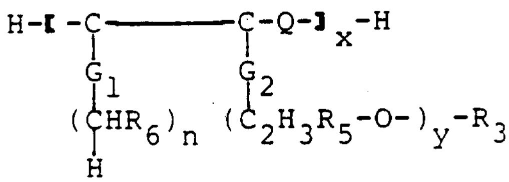

(I')

in der alle Gruppen die obige Bedeutung haben,
zur Herstellung von pharmazeutischen Zubereitungen.

10. Verwendung von Polymeren der Formel I"

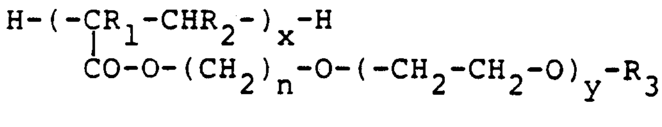

(I'')

in der $R_1$, $R_2$, $R_3$, x, n, und y die vorstehende Bedeutung haben,
zur Herstellung von pharmazeutischen Zubereitungen von in Wasser schwer löslichen Wirkstoffen.

## Claims

1. Pharmaceutical formulations containing a pharmaceutical active material sparingly soluble in water and a solubiliser, characterised in that the solubiliser is a polymer of the general formula I

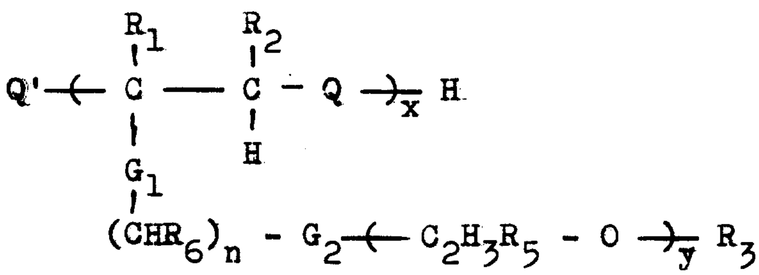

(I)

in which $R_1$, $R_2$, $R_5$ and $R_6$ are the same or different and represent hydrogen or a methyl or ethyl group,

Q represents a valency, oxygen or an ester or amide bridge and Q' signifies hydrogen when Q represents a valency or oxygen and is a hydroxyl or amino group when Q represents an ester or amide bridge, x is a whole number of 3 - 50, preferably 5 - 40 when Q is a valency or oxygen and a whole number of 3 - 1000, preferably 50 - 100 when Q is an ester or amide function,
$G_1$ and $G_2$ is a valency, oxygen or an ester or amide group, whereby the two groups can be the same or different,
n is a whole number of 4 - 44, preferably 12 - 16,
y is a whole number of 2 - 50, preferably 10 - 40 and
$R_3$ is hydrogen or a lower alkyl group with 1 - 6 C-atoms, or the polymer has the formula I'

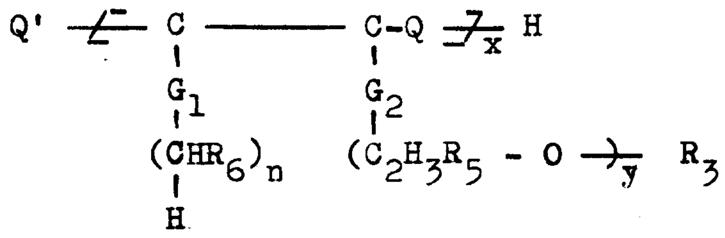

(I')

in which all groups have the above meaning.

2. Pharmaceutical formulation according to claim 1, characterised in that the polymer is a polymer of the formula I",

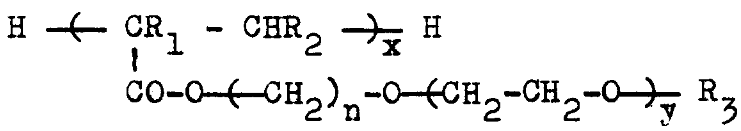

(I")

in which $R_1$, $R_2$, $R_3$, x, n and y have the above-given meaning.

3. Pharmaceutical formulation according to claim 2, characterised in that $R_1$, $R_3$ and x have the above-given meaning, $R_2$ is hydrogen, n is equal to 10 and y is equal to 8.

4. Pharmaceutical formulation according to claims 1 to 3, characterised in that x is equal to 7.

5. Pharmaceutical formulation according to claim 1, characterised in that the polymer is a polymer of the formula I"

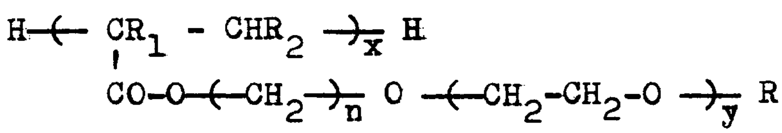

(I")

in which $R_1$, $R_2$, $R_5$ and $R_6$ are the same or different and represent hydrogen or a methyl or ethyl group,

x is a whole number of 3 - 50, preferably 5 - 40, when Q is a valency or oxygen and a whole number of 3 - 1000, preferably 50 - 100, when Q is an ester or amide function, $G_1$ and $G_2$ is a valency, oxygen or an ester or amide group, whereby the two groups can be the same or different, n is a whole number of 4 - 44, preferably 12 - 16, y is a whole number of 2 - 50, preferably 10 - 40 and $R_3$ is hydrogen or a lower alkyl group with 1 - 6 C-atoms, or the polymer has the formula I'"

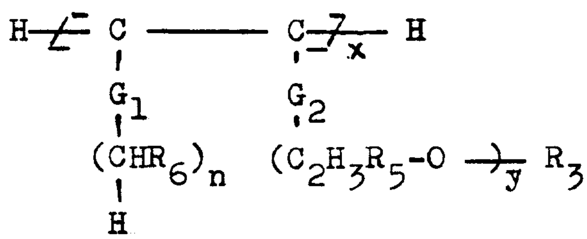

(I'")

in which all groups have the above meaning.

6. Pharmaceutical formulation according to claim 5, characterised in that $R_1$, $R_3$ and x have the above meaning, $R_2$ is hydrogen, n is equal to 10 and y is equal to 8.

7. Pharmaceutical formulation according to claims 5 and 6, characterised in that x is equal to 7.

8. Pharmaceutical formulation, characterised in that the solubiliser contains 11,14,17,20,23,26,29,32,35-nonaoxahexatriacontan-1-ol or its tetrahydropyranyl ether.

9. Use of polymers of the formula I

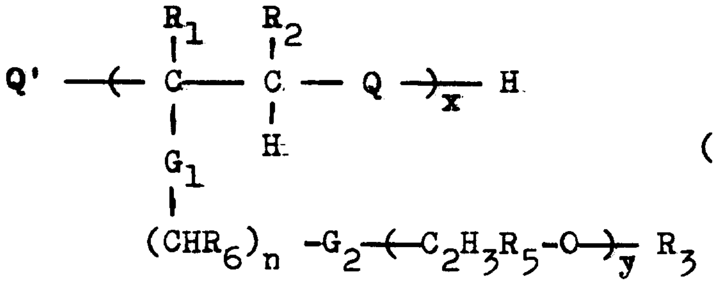

(I)

in which $R_1$, $R_2$, $R_5$ and $R_6$ are the same or different and represent hydrogen or a methyl or ethyl group,

Q represents a valency, oxygen or a an ester or amide bridge and Q' signifies hydrogen when Q represents a valency or oxygen and is a hydroxyl or amino group when Q represents an ester or amide bridge, x is a whole number of 3 - 50, preferably 5 - 40, when Q is a valency or oxygen and a whole number of 3 - 1000, preferably 50 - 100, when Q is an ester or amide function, $G_1$ and $G_2$ is a valency, oxygen or an ester or amide group, whereby the two groups can be the same or different, n is a whole number of 4 - 44, preferably 12 - 16, y is a whole number of 2 - 50, preferably 10 - 40 and $R_3$ is hydrogen or a lower alkyl group with 1 - 6 C-atoms, or the polymer has the formula I'

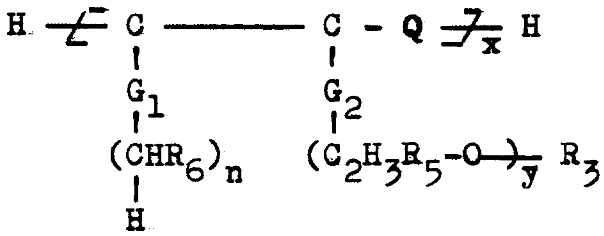

(I')

in which all groups have the above meaning, for the production of pharmaceutical formulations.

10. Use of polymers of the formula I"

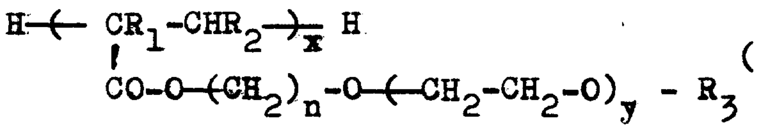

(I")

in which $R_1$, $R_2$, $R_3$, x, n and y have the above meaning, for the production of pharmaceutical formulations of active materials sparingly soluble in water.

**Revendications**

1. Formulation de préparations pharmaceutiques contenant une substance pharmaceutique active peu soluble dans l'eau et un agent de solubilisation, caractérisée en ce que l'agent de solubilisation est un polymère répondant à la formule générale I,

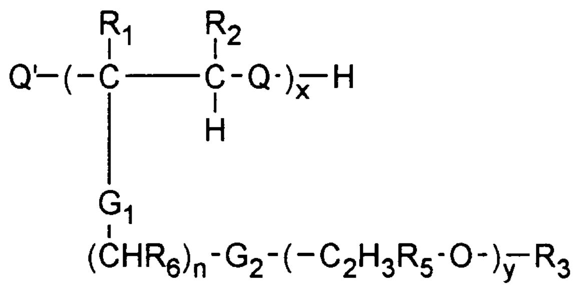

(I)

dans laquelle $R_1$, $R_2$, $R_5$ et $R_6$ sont identiques ou différents et représentent un atome d'hydrogène ou un groupe méthyle ou éthyle,

Q représente une valence, un atome d'oxygène ou un pont ester ou amide et Q' signifie un atome d'hydrogène, lorsque Q représente une valence ou un atome d'oxygène, et un groupe hydroxyle, respectivement un groupe amino, lorsque Q représente un pont ester, respectivement amide,
x représente un nombre entier de 3 à 50, de préférence de 5 à 40, lorsque Q représente une valence ou un atome d'oxygène, et un nombre entier de 3 à 1000, de préférence de 50 à 100, lorsque Q représente une fonction ester ou amide,
$G_1$ et $G_2$ représentent une valence, un atome d'oxygène ou un groupe ester ou amide, les deux groupes pouvant être identiques ou différents, n représente un nombre entier de 4 à 44, de préférence de 12 à 16,
y représente un nombre entier de 2 à 50, de préférence de 10 à 40, et
$R_3$ représente un atome d'hydrogène ou un groupe alkyle inférieur avec 1 à 6 atomes de carbone,

ou en ce que le polymère répond à la formule I'

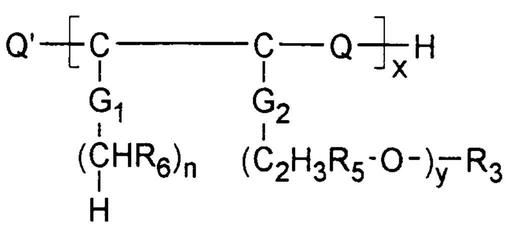

(I')

dans laquelle tous les groupes ont la signification susmentionnée.

2. Préparation pharmaceutique selon la revendication 1, caractérisée en ce que le polymère est un polymère répondant à la formule I",

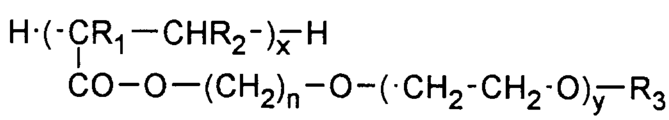

(I")

dans laquelle $R_1$, $R_2$, $R_3$, x, n et y ont la signification précédente.

3. Préparation pharmaceutique selon la revendication 2, caractérisée en ce que $R_1$, $R_3$, et x ont la signification précédente, $R_2$ représente un atome d'hydrogène, n vaut 10 et y vaut 8.

4. Préparation pharmaceutique selon une des revendications 1 à 3, caractérisée en ce que x vaut 7.

5. Préparation pharmaceutique selon la revendication 1, caractérisée en ce que le polymère est un polymère répondant à la formule I",

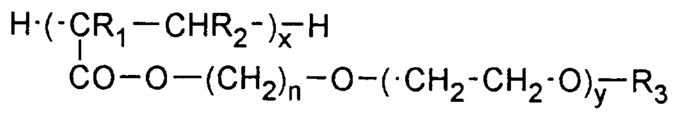

(I'')

dans laquelle $R_1$, $R_2$, $R_5$ et $R_6$ sont identiques ou différents et représentent un atome d'hydrogène ou un groupe méthyle ou éthyle,

x représente un nombre entier de 3 à 50, de préférence de 5 à 40, lorsque Q représente une valence ou un atome d'oxygène, et un nombre entier de 3 à 1000, de préférence de 50 à 100, lorsque Q représente une fonction ester ou amide,

$G_1$ et $G_2$ représentent une valence, un atome d'oxygène ou un groupe ester ou amide, les deux groupes pouvant être identiques ou différents, n représente un nombre entier de 4 à 44, de préférence de 12 à 16,

y représente un nombre entier de 2 à 50, de préférence de 10 à 40, et

$R_3$ représente un atome d'hydrogène ou un groupe alkyle inférieur avec 1 à 6 atomes de carbone,

ou en ce que le polymère répond à la formule I'''

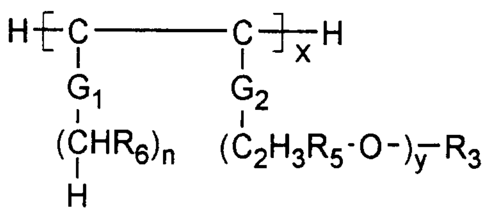

(I''')

dans laquelle tous les groupes ont la signification susmentionnée.

6. Préparation pharmaceutique selon la revendication 5, caractérisée en ce que $R_1$, $R_3$, et x ont la signification précédente, $R_2$ représente un atome d'hydrogène, n vaut 10 et y vaut 8.

7. Préparation pharmaceutique selon les revendications 5 et 6, caractérisée en ce que x vaut 7.

8. Préparation pharmaceutique caractérisée en ce que l'agent de solubilisation contient le 11,14,17,20,23,26,29,32,35-nonaoxahexatriacontan-1-ol ou son éther tétrahydropyranylique.

9. Utilisation de polymères répondant à la formule I

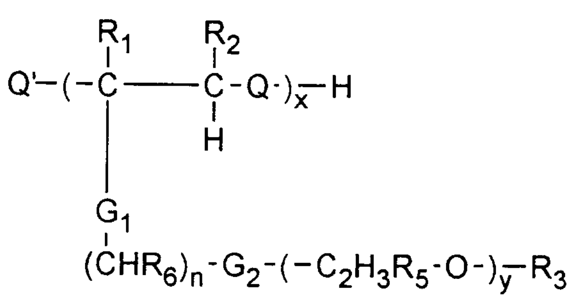

(I)

dans laquelle $R_1$, $R_2$, $R_5$ et $R_6$ sont identiques ou différents et représentent un atome d'hydrogène ou un groupe méthyle ou éthyle,

Q représente une valence, un atome d'oxygène ou un pont ester ou amide et Q' signifie un atome d'hydrogène, lorsque Q représente une valence ou un atome d'oxygène, et un groupe hydroxyle, respectivement un groupe amino, lorsque Q représente un pont ester, respectivement amide,

x représente un nombre entier de 3 à 50, de préférence de 5 à 40, lorsque Q représente une valence ou un atome d'oxygène, et un nombre entier de 3 à 1000, de préférence de 50 à 100, lorsque Q représente une fonction ester ou amide,

$G_1$ et $G_2$ représentent une valence, un atome d'oxygène ou un groupe ester ou amide, les deux groupes pouvant être identiques ou différents, n représente un nombre entier de 4 à 44, de préférence de 12 à 16,

y représente un nombre entier de 2 à 50, de préférence de 10 à 40, et

$R_3$ représente un atome d'hydrogène ou un groupe alkyle inférieur avec 1 à 6 atomes de carbone,

ou en ce que le polymère répond à la formule I'

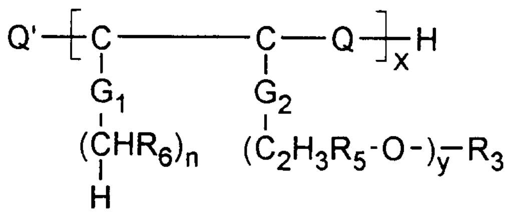

(I')

dans laquelle tous les groupes ont la signification susmentionnée, pour la production de préparations pharmaceutiques.

**10.** Utilisation de polymères répondant à la formule I"

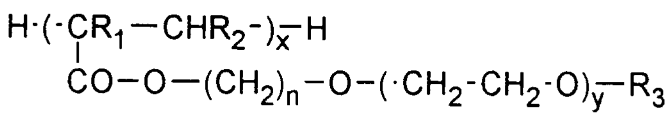

(I")

dans laquelle $R_1$, $R_2$, $R_3$, x, n et y ont la signification précédente, pour la production de préparations pharmaceutiques de substances actives peu solubles dans l'eau.